## Europäisches Patentamt

## European Patent Office

## Office européen des brevets

(11) Veröffentlichungsnummer: **0 110 411**
**B1**

(12) # EUROPÄISCHE PATENTSCHRIFT

(45) Veröffentlichungstag der Patentschrift:
09.04.86

(21) Anmeldenummer: **83112078.7**

(22) Anmeldetag: **01.12.83**

(51) Int. Cl.⁴: **C 07 B 61/00**, B 01 J 12/00 //
C07D213/12, C07D231/06,
C07D233/06, C07D239/06

(54) Verfahren zur Durchführung von organischen Kondensationsreaktionen.

(30) Priorität: **07.12.82 DE 3245109**

(43) Veröffentlichungstag der Anmeldung:
**13.06.84 Patentblatt 84/24**

(45) Bekanntmachung des Hinweises auf die Patenterteilung:
**09.04.86 Patentblatt 86/15**

(84) Benannte Vertragsstaaten:
**AT BE CH DE FR GB LI NL**

(56) Entgegenhaltungen:
**CH - A - 214 894**
**FR - A - 847 617**
**GB - A - 1 298 687**

(73) Patentinhaber: **BASF Aktiengesellschaft,
Carl-Bosch-Strasse 38, D-6700 Ludwigshafen (DE)**

(72) Erfinder: **Dockner, Toni, Dr., Grossgasse 6,
D-6701 Meckenheim (DE)**
Erfinder: **Krug, Herbert, Mussbacher Strasse 49,
D-6700 Ludwigshafen (DE)**

LIBER, STOCKHOLM 1986

## Beschreibung

Die vorliegende Erfindung betrifft ein Verfahren zur Durchführung von organischen Kondensationsreaktionen bei erhöhten Temperaturen in einem mit einem Mineralöl gefüllten Reaktor.

Es ist bekannt, organische Kondensationsreaktionen bei erhöhten Temperaturen in der Gasphase durchzuführen, wobei in der Regel Temperaturen von 150°C bis 550°C angewendet werden. Bei den relativ hohen Temperaturen kommt es leicht zu Abscheidungen von Crackprodukten in den Reaktoren und an anderen Apparaten wie Wärmetauschern sowie bei Verwendung von Katalysatoren an den Katalysatoroberflächen. Diese Abscheidungen führen häufig zu Betriebsunterbrechungen, bei denen z.B. der Reaktor und die Wärmetauscherflächen gereinigt werden und der Katalysator gewechselt oder regeneriert werden müssen.

Es bestand daher Bedarf nach einem Verfahren zur Durchführung von organischen Kondensationsreaktionen, bei dem die Nachteile der bekannten Verfahren vermieden werden können.

Es wurde nun ein vorteilhaftes Verfahren gefunden zur Durchführung von organischen Kondensationsreaktionen bei erhöhten Temperaturen, welches dadurch gekennzeichnet ist, daß man den Ausgangsstoff oder die Ausgangsstoffe gasförmig am Boden eines mit Mineralöl mit einem Siedepunkt von mindestens 150°C gefüllten Reaktors zuführt bzw. flüssig am Boden des Reaktors zuführt und dabei das Mineralöl bei einer Temperatur hält, die mindestens so hoch ist wie die den sich am Boden des Reaktors einstellenden Partialdrucken der flüssig zugeführten Ausgangsstoffe entsprechenden Siedepunkte der flüssig zugeführten Ausgangsstoffe, und das Reaktionsprodukt durch Destillation erhalten wird, z.B., daß man das Reaktionsprodukt gasförmig am Kopf des Reaktors abzieht und kondensiert.

Nach dem neuen Verfahren lassen sich Gasphasenreaktionen durchführen, ohne daß es zu Abscheidungen von Zersetzungsprodukten in den Reaktoren kommt. Besonders bei katalytischen Kondensationsreaktionen kann der Katalysator dem Mineralöl als löslicher oder als unlöslicher Katalysator zugesetzt werden, so daß anders als bei den konventionellen Gasphasenreaktionen die Belegung der Oberfläche des Katalysators und dessen Inaktivierung und die sich daraus aufgrund des erforderlichen Katalysatorwechsels und der Katalysatorregenerierung ergebenden häufigen Betriebsunterbrechungen vermieden werden können.

Organische Kondensationsreaktionen, die nach dem neuen Verfahren durchgeführt werden, sind z.B. Reaktionen, bei denen aus organischen Ausgangsstoffen unter Abspaltung von $H_2O$ oder $NH_3$ und Knüpfung von Kohlenstoff-Stickstoffbindungen sowie Kohlenstoff-Kohlenstoffbindungen monomere Verbindungen entstehen. Neben der Kondensationsreaktion können bei der Bildung der Reaktionsprodukte auch noch andere Reaktionen, z.B. Additionsreaktionen, beteiligt sein. Dabei sind an der Reaktion im allgemeinen 1 bis 4 Ausgangsstoffe beteiligt. Vorzugsweise handelt es sich bei den Reaktionsprodukten um heterocyclische Verbindungen, insbesondere um 5- oder 6-gliedrige Heterocyclen. Ceeignete organische Kondensationsreaktionen sind beispielsweise die Umsetzung von

- Hydrazin mit Acrolein zu Δ2-Pyrazolin,
- N',N'-Diformyl-1,2-diaminoethan zu Δ2-Imidazolin
- N,N'-Diformyl-1,2-diaminopropan zu 4-Methyl-Δ2-imidazolin.
- Acrolein mit Ammoniak zu ß-Picolin
- N,N'-Diformyl-1,3-diaminopropan zu 1,2,3,4-Tetrahydropyrimidin.
- Essigsäure mit Ethylendiamin zu 2-Methylimidazolin

Das erfindungsgemäße Verfahren wird in der Weise durchgeführt, daß man den Ausgangsstoff oder die Ausgangsstoffe gasförmig am Boden eines mit Mineralöl mit einem Siedepunkt von mindestens 150°C, vorzugsweise mindestens 200°C, insbesondere mindestens 300°C, gefüllten Reaktors zuführt bzw. flüssig am Boden des Reaktors zuführt und dabei das Mineralöl bei einer Temperatur hält, die mindestens so hoch ist wie die den sich am Boden des Reaktors einstellenden Partialdrucken der flüssig zugeführten Ausgangsstoffe entsprechenden Siedepunkte der flüssig zugeführten Ausgangsstoffe, und das Reaktionsprodukt gasförmig am Kopf des Reaktors abzieht. Als Mineralöl kommen beispielsweise hochsiedende Kohlenwasserstoffe oder Kohlenwasserstoff-Fraktionen wie Gasöl, Heizöl, geschmolzenes Paraffinwachs, ein aromatisches Kohlenwasserstofföl in Betracht. Vorteilhaft wird Vakuumgasöl mit einem Siedepunkt von mindestens 350°C, insbesondere mit einem Siedebereich zwischen 350°C und 550°C verwendet.

Als Reaktoren sind für die Kondensationsreaktion z.B. Rührkessel geeignet. Vorteilhaft werden jedoch für das neue Verahren senkrecht angeordnete zylindrische Reaktoren wie Glockenbodenkolonnen, Blasensäulen oder Füllkörperkolonnen verwendet. Bei der Reaktion mehrerer Ausgangsstoffe ist es auch möglich, einen oder mehrere der Ausgangsstoffe außerhalb des Reaktors zu verdampfen und gasförmig am Boden des Reaktors einzuleiten und den übrigen oder die übrigen Ausgangsstoffe flüssig am Boden des Reaktors einzuleiten und dort zu verdampfen. Es kann vorteilhaft sein, den verdampften Ausgangsstoff bzw. die verdampften Ausgangsstoffe mit einem inerten Gas zu verdünnen. Geeignete inerte Gase sind z.B. Wasserdampf, Kohlendioxid und vorzugsweise Stickstoff.

Die Kondensationsreaktion wird im allgemeinen bei Temperaturen von 100°C bis 500°C, vorzugsweise 100°C bis 400°C, insbesondere 150°C bis 350°C durchgeführt. Im allgemeinen werden Atmosphärendruck oder erhöhter Druck z.B. bis zu einem Gesamtdruck von 10 bar angewendet. Es ist jedoch auch möglich, bei vermindertem Druck z.B. bis herunter zu 100 mbar zu arbeiten.

Die Kondensationsreaktion kann je nach Art der Reaktion mit oder ohne Kondensationskatalysatoren durchgeführt werden. Bei Verwendung von Katalysatoren können sowohl im Mineralöl unlösliche als auch im Mineralöl lösliche Katalysatoren verwendet werden. Die unlöslichen Katalysatoren werden zweckmäßig in im Mineralöl suspendierter Form verwendet. Vorzugsweise werden jedoch im Mineralöl gelöste Katalysatoren verwendet. Geeignete Kondensationskatalysatoren sind beispielsweise saure Katalysatoren, z.B. saure Ionenaustauscher, aromatische Sulfonsäuren wie Benzolsulfonsäure, Toluolsulfonsäure, Dodecylbenzolsulfonsäure, o-Alkylphosphorsäuren, Phenole wie Alkylphenole, Pikrinsäure oder basische Katalysatoren, z.B. primäre, sekundäre oder tertiäre Amine wie Tridecylamin, basische Ionenaustauscher. Die im Mineralöl löslichen Katalysatoren werden dem Mineralöl im allgemeinen in Mengen von 0,01 bis 25 Gew.%, vorzugsweise 0,1 bis 10 Gew.%, insbesondere 0,5 bis 5 Gew.%, bezogen auf das Mineralöl, zugesetzt.

Die Reaktionsprodukte werden gasförmig am Kopf des Reaktors abgezogen. Anschließend werden die gasförmigen Reaktionsprodukte zweckmäßig kondensiert. An die Kondensation kann noch eine Reinigungsstufe, z.B. eine Destillation oder Fraktionierung angeschlossen werden. Es ist jedoch auch möglich, die gasförmigen Reaktionsprodukte unmittelbar einem zweiten Reaktionsschritt zuzuführen.

Das neue Verfahren kann diskontinuierlich oder kontinuierlich durchgeführt werden, wobei jedoch die kontinuierliche Arbeitsweise bevorzugt wird. Bei der kontinuierlichen Arbeitsweise kann es vorteilhaft sein, das Mineralöl kontinuierlich zuzuführen und abzuziehen, beispielsweise wenn es sich um eine Reaktion handelt, bei der Crackprodukte gebildet werden. iese Crackprodukte werden dabei mit dem Mineralöl kontinuierlich aus dem Reaktor ausgeschleust. Eine Aufarbeitung und Rückführung des abgezogenen Mineralöls ist in der Regel nicht wirtschaftlich, da das Mineralöl, z.B. als Heizöl oder Vakuumgasöl in der Regel wohlfeil zur Verfügung steht.

Man wird daher zweckmäßig das abgezogene, Crackprodukte enthaltende Mineralöl verbrennen und dem Reaktor frisches Mineralöl zuführen.

Die folgenden Beispiele veranschaulichen die Erfindung.

## Beispiel 1

155 g Hydrazinhydrat werden (vgl. die Fig.) pro Stunde aus einem Vorrats-gefäß 1 über eine Dosierpumpe 2 nach Zugabe von 50 Nl pro Stunde Stickstoff über Leitung 3 in einen auf 120°C erhitzten Verdampfer 4 dosiert und von dort gasförmig über eine Zweistoffdüse 5 in einen mit 1300 g Vakuumgasöl mit einem Siedepunkt von mindestens 350°C gefüllten und auf 170°C erhitzten Reaktor 6 geleitet. Gleichzeitig werden 174 g Acrolein pro Stunde aus dem Vorratsgefäß 7 über Dosierpumpe 8 nach Zugabe von 50 Nl pro Stunde Stickstoff über Leitung 9 im Verdampfer 10 bei 70°C verdampft und von dort ebenfalls gasförmig über die Zweistoffdüse 5 in den Reaktor 6 geleitet. Der Reaktor ist ein mit Doppelmantel versehenes Glasrohr mit einer Länge von 1300 mm und einem inneren Durchmesser von 60 mm. Das Vakuumgasöl enthält 1 Gew.% Dodecylbenzolsulfonsäure als Katalysator. Die den Reaktor über Leitung 11 verlassenden Dämpfe werden kondensiert und in einem Glaskolben gesammelt. Man erhält stündlich 321 g Produkt, das 55,7 Gew.% $\Delta$ 2-Pyrazolin enthält, entsprechend einer Ausbeute bezogen auf Hydrazin von 82,2 %.

## Beispiel 2

28 g N,N'-Diformyl-1,2-diaminoethan werden pro Stunde aus einem Vorrats-gefäß in einen auf 250°C erhitzten horizontalen Quarzverdampfer dosiert und der Dampf mit 60 Nl pro Stunde Stickstoff in einen auf 200°C erhitzten Reaktor geleitet. Der Reaktor ist ein vertikal auf dem Verdampfer sitzendes elektrisch beheiztes Quarzrohr (Länge = 500 mm, innerer Durchmesser = 60 mm), das nach unten mit einer eingeschmolzenen Quarzfritte abgeschlossen ist. Das Quarzrohr ist mit 300 g Vakuumgasöl mit einem Siedepunkt von mindestens 350°C gefüllt. Die den Reaktor verlassenden Dämpfe werden kondensiert und fraktioniert destilliert. Man erhält stündlich 30,7 g (90 % d. Theorie) $\Delta$2-Imidazolin vom Kp. 105°C (bei 32 mbar). Der Umsatz ist praktisch quantitativ.

## Beispiel 3

In der in Beispiel 2 beschriebenen Apparatur werden 300 g Vakuumgasöl mit einem Siedepunkt von mindestens 350°C mit 1 % Tridecylamin als Katalysator eingesetzt. Stündlich werden 35 g N,N'-Diformyl-1,2-diaminopropan bei 250°C mit 60 Nl Stickstoff erhitzt und das erhaltene gasförmige Gemisch wird dem Reaktor, in dem eine Reaktionstemperatur von 190°C aufrechterhalten wird, am Boden zugeführt. Das am Kopf des Reaktors gasförmig abgezogene Reaktionsprodukt wird kondensiert und anschließend fraktioniert destilliert. Man erhält stündlich 41,7 g (92 % d.Th.) 4-(5)-Methyl-2-imidazolin vom Kp. 94°C (bei 20 mbar).

**Beispiel 4**

In der in Beispiel 2 beschriebenen Apparatur werden 300 g Vakuumgasöl mit einem Siedebereich von 375°C bis 530°C eingesetzt. Stündlich werden 20 g N,N'-Diformyl-1,3-diaminopropan bei 180°C mit 70 bis 80 Nl pro Stunde Stickstoff erhitzt und das erhaltene gasförmige Gemisch wird dem Reaktor, in dem eine Reaktionstemperatur von 200°C aufrechterhalten wird, über eine am Boden angeordnete Fritte zugeführt. Das gasförmige am Kopf des Reaktors abgezogene und anschließend kondensierte Reaktionsprodukt enthält 9,5 g/h 1,2,3,4-Tetrahydropyrimidin. Dies entspricht einer Ausbeute von 75 % der Theorie.

**Beispiel 5**

56 g/h Acrolein werden aus einem Vorratsgefäß über eine Dosierpumpe nach Zugabe von 20 Nl/h Stickstoff, gemessen über ein Rotometer, über ein Tauchrohr am Boden eines auf 250°C beheizten Rührkolbens, der 1,3 kg Vakuumgasöl mit einem Siedebereich von 375°C bis 530°C mit 5 Gew.% Dodecylbenzolsulfonsäure als Katalysator enthält, zugeführt. Gleichzeitig werden 10 Nl/h Ammoniak über ein zweites Tauchrohr ebenfalls am Boden des Rührkolbens zugeführt. Das den Rührkolben gasförmig verlassende Reaktionsgemisch wird über einen bei 150°C gehaltenen Kondensator geleitet um mitgerissenes Vakuumgasöl zurückzuführen und anschließend kondensiert. Man erhält stündlich 49 g ß-Picolin (53 % der Theorie) und 18 g Pyridin (22 % der Theorie).

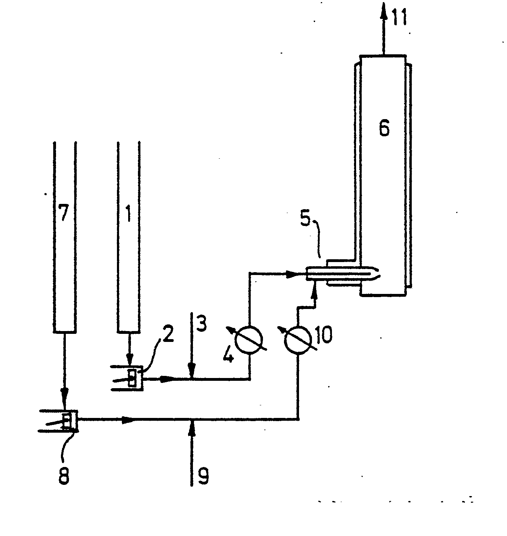

**Patentansprüche**

1. Verfahren zur Durchführung von organischen Kondensationsreaktionen bei erhöhten Temperaturen, dadurch gekennzeichnet, daß man den Ausgangsstoff oder die Ausgangsstoffe gasförmig am Boden eines mit Mineralöl mit einem Siedepunkt von mindestens 150°C gefüllten Reaktors zuführt bzw. flüssig am Boden des Reaktors zuführt und dabei das Mineralöl bei einer Temperatur hält, die mindestens so hoch ist, daß die am Boden des Reaktors flüssig zugeführten Ausgangsstoffe verdampfen und das Reaktionsprodukt durch Abdestillieren gewinnt.

2. Derfahren gemäß Anspruch 1, dadurch gekennzeichnet, daß man das Reaktionsprodukt gasförmig am Kopf des Reaktors abzieht.

3. Verfahren gemäß Anspruch 1, dadurch gekennzeichnet, daß man die Ausgangsstoffe in dem Mineralöl umsetzt, das Mineralöl bei Anreicherung mit Nebenprodukten erneuert und das mit Nebenprodukten angereicherte Mineralöl abzieht.

4. Verfahren nach Anspruch 3, dadurch gekennzeichnet, daß man das mit Nebenprodukt angereicherte Mineralöl einer Verfeuerung zuführt.

5. Verfahren nach Anspruch 1, durch gekennzeichnetdaß als Mineralöl hochsiedende Kohlenwasserstoff-Fraktionen verwendet werden.

6. Verfahren nach Anspruch 1, dadurch gekennzeichnet, daß man als Mineralöl Vakuumgasöl mit einem Siedepunkt von mindestens 350°C verwendet.

7. Verfahren nach Anspruch 1, dadurch gekennzeichnet, daß man die Kondensationsreaktion in einem zylindrischen Reaktor durchführt.

8. Verfahren nach Anspruch 1, dadurch gekennzeichnet, daß man dem Mineralöl lösliche oder unlösliche Katalysatoren zusetzt.

9. Verfahren nach Anspruch 1, dadurch gekennzeichnet, daß man das Verfahren kontinuierlich durchführt.

10. Verfahren nach Anspruch 1, dadurch gekennzeichnet, daß man das Mineralöl kontinuierlich zuführt und abzieht.

11. Verfahren nach Anspruch 1, dadurch gekennzeichnet, daß man im Reaktor eine Reaktionstemperatur aufrechterhält, die mindestens so hoch ist wie die den sich im Reaktor einstellenden Partialdrucken der Ausgangsstoffe und Reaktionsprodukte entsprechenden Siedepunkte der Ausgangsstoffe und Reaktionsprodukte.

**Claims**

1. A method of carrying out organic condensation reactions at elevated temperatures, wherein the starting material or materials are fed in, in gaseous form, at the bottom of a reactor charged with mineral oil having a boiling point of not less than 150°C, or are fed in, in liquid form, at the bottom of the reactor, and the mineral oil is kept at a temperature which is sufficiently high for the starting materials introduced as liquids at the bottom of the reactor to vaporize, and the product is obtained by distillation.

2. A method as claimed in claim 1, wherein the product is taken off in gaseous form at the top of the reactor.

3. A method as claimed in claim 1, wherein the starting materials are reacted in the mineral oil, fresh mineral oil is introduced where enrichment with byproducts occurs, and the mineral oil enriched with byproducts is removed.

4. A method as claimed in claim 3, wherein the mineral oil enriched with by-products is fed to a combustion stage.

5. A method as claimed in claim 1, wherein the mineral oil used is a high-boiling hydrocarbon fraction.

6. A method as claimed in claim 1, wherein the mineral oil used is a vacuum gas oil having a boiling point of not less than 350°C.

7. A method as claimed in claim 1, wherein the condensation reaction is carried out in a cylindrical reactor.

8. A method as claimed in claim 1, wherein a soluble or insoluble catalyst is added to the mineral oil.

9. A method as claimed in claim 1, which is carried out continuously.

10. A method as claimed in claim 1, wherein the mineral oil is continuously fed in and withdrawn.

11. A method as claimed in claim 1, wherein the reaction temperature maintained in the reactor is no lower than the boiling points, of the starting materials and products, which correspond to their partial pressures in the reactor.

## Revendications

1. Procédé de réalisation de réactions de condensation organiques à des températures accrues, caractérisé en ce que l'on introduit le composé de départ ou les composes de départ à l'état gazeux à la base d'un réacteur rempli d'une huile minérale d'un point d'ebullition au moins egal à 150°C, ou on introduit le ou les composés de depart à l'état liquide à la base du réacteur en maintenant l'huile minérale à une température au moins suffisante pour vaporiser les composés de départ introduits à l'état liquide, le produit réactionnel étant séparé par distillation.

2. Procédé suivant la revendication 1, caractérisé en ce que le produit réactionnel est soutiré à l'état gazeux au sommet du réacteur.

3. Procédé suivant la revendication 1, caractérisé en ce que l'on fait réagir les composés de depart dans l' huile minérale et on remplace l'huile minérale enrichie en sous-produits par de l'huile fraîche.

4. Procédé suivant la revendication 3, caractérisé en ce que l'huile minérale chargée de sous-produits est utilisée comme combustible.

5. Procédé suivant la revendication 1, caractérisé en ce que l'huile minérale est choisie parmi les coupes d'hydrocarbures à point d'ébullition élevé.

6. Procédé suivant la revendication 1, caractérisé en ce que l'huile minérale est un gasoil vacuum avec un point d'ébullition au moins égale à 350°C.

7. Procédé suivant la revendication 1, caractérisé en ce que la réaction de condensation est réalisée dans un réacteur cylindrique.

8. Procédé suivant la revendication 1, caractérisé en ce que l'on ajoute à l'huile minérale un catalyseur soluble ou un catalyseur insoluble.

9. Procédé suivant la revendication 1, caractérisé en ce qu'il est réalisé en continu.

10. Procédé suivant la revendication 1, caractérisé en ce que l'huile minérale est ajoutée et soutirée en continu.

11. Procédé suivant la revendication 1, caractérisé en ce que l'on maintient dans le réacteur une température au moins égale aux températures d'ébullition des produits de départ et des produits réactionnels aux tensions partielles des produits de départ et des produits réactionnels, qui s'établissent dans le réacteur.